(19) Europäisches Patentamt
European Patent Office
Office européen des brevets

(11) EP 1 393 752 A1

(12) **EUROPEAN PATENT APPLICATION**

(43) Date of publication:
**03.03.2004 Bulletin 2004/10**

(51) Int Cl.⁷: $A61L\ 9/01$, $C11B\ 9/00$

(21) Application number: **03018903.9**

(22) Date of filing: **20.08.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **26.08.2002 US 227962**

(71) Applicant: **Symrise GmbH & Co. KG**
**37603 Holzminden (DE)**

(72) Inventors:
• **Costa, Jill**
**Goshen New York 10924 (US)**
• **Aikens, Patricia**
**Bronx New York 10471 (US)**

(74) Representative: **Eisenführ, Speiser & Partner**
**Patentanwälte Rechtsanwälte**
**Postfach 10 60 78**
**28060 Bremen (DE)**

(54) **Malodor suppression by fragrance composition**

(57) A method and composition for suppressing residential malodor, said composition comprising a fragrance having at least one perfume material which satisfies one of the following criteria, (1) contains a phenyl ring moiety and has an air diffusion coefficient of > 5.7, or (2) contains a C-5 ring moiety, which also contains at least 1 carbon which is sp2 hybridized, and has an air diffusion coefficient of > 4.4.

**EP 1 393 752 A1**

## Description

This invention concerns a fragrance composition for masking residential malodors. More specifically, the invention concerns the use of a fragrance composition having a perfume with specific properties to mask malodors such as cat litter odor, trash and garbage odor, urine odor and kitchen odors.

Malodorous smells exist in many environments. Such odors are created in industrial, commercial, and residential environments. In industrial environments, the odors are often created by processes involving the use of chemicals. In commercial and residential environments, malodorous odors can be generated by, for example, waste product, trash receptacles, cat litter, and food handling and processing.

Foods such as fish, onions, garlic or other spices, cooking odors, smoke, tobacco, trash and pet litter, are just a few of the common environmental sources of malodors in daily life.

Numerous attempts have been made to conceal malodors odors through the use of deodorizing compositions. A number of types of agents for odor treatment are utilized. Broadly, such agents fall into two categories. These include neutralizers and maskers.

The odor neutralizing agents function to interact with the malodors and chemically neutralize them. The prior art teaches the use of oxidizing agents such as oxygen bleaches, chlorine; chlorinated materials such as sodium hypochlorite, chlorine dioxide; potassium permanganate; reducing agents such as sodium bisulfite to reduce malodor. But, unfortunately, these neutralizing agents are flammable, explosive, corrosive, or toxic.

The odor masking agents function to intentionally conceal odors by the addition of another. Odor masking has been accomplished by using perfumes, fragrances, colognes, etc.

In general, the masking agents suppress the malodor by:

   providing a more pleasing aroma using large quantities of a fragrance; or
   providing a fragrance that blends with the malodor to provide a different and more desirable aroma.

Unfortunately, in both instances, a large amount of fragrance must be utilized, which in itself often proves to be offensive. Furthermore, the malodor is usually still detectable at the levels of masking fragrances that are reasonably tolerable.

United States Patent No. 4,009,253 entitled "4-Cyclohexyl-4-methyl-2-pentanone Useful as a Malodor Counteractant" to Schleppnik et al. discloses compositions comprising 4-cyclohexyl-4-methyl-2-pentanone to counteract malodors.

United States Patent No. 4,622,221 entitled "Method, Compositions and Compounds, Useful in Room Fresheners Employing Cyclohexyl Alcohol and Ester Derivatives" to Schleppnik discloses that specified cyclohexyl compound can be used as malodor counteractant.

Unfortunately, the use of cyclohexyl in animal products, such as litter, often produces a disliked smell that prevents household pets from using such products and avoiding the use of the litter. Further, these products are effective only over a limited period of time.

United States Patent No. 5,683,979 entitled "Malodor Counteractant Composition and Process for Using Same" to Schreck et al. discloses a composition for counteracting malodor such as pet urine, hair dye, human urine, feces, tobacco, cooking odors, garbage, and mildew. The composition comprises a fragrance in an amount from about 0.6 up to about 25% by weight of the final product; the fragrance consisting essentially of musk, citrus, and mint.

A problem presented by the Schreck et al. reference is that the masking effect is an additive effect, and so the total odor level in the superficies increases by the application of the masking composition. Even though the fragrances used in the composition may, in small quantities, be very pleasant, the total odor level in the superficies at concentrations sufficient to achieve moderate masking of the malodor may itself be offensive.

Recently, compositions containing antibacterial and antifungal agents have been used in the art to regulate the malodor produced by microorganisms found on the surface to which the composition is directed. Many skin deodorant products use this technology. TA problem presented by these compositions is that they are not effective on odors that do not come from bacterial sources, such as tobacco or food odors.

Thus, there remains a need for a composition for masking residential malodors that: 1) is safe and effective for use in the residence; 2) can mask malodors initially as well as over time; 3) can absorb a broad spectrum of malodors; and 4) has a smell that is pleasant to the user.

It is an object of the present invention to provide a fragrance composition for masking residential malodors that is safe and effective for use in the residence.

It is yet another object of the present invention to provide a fragrance composition for masking residential malodors that can mask malodor initially as well as over time.

It is yet another object of the present invention to provide a fragrance composition that can absorb a broad spectrum of malodors.

It is yet another object of the present invention to provide a fragrance composition that provides a pleasant smell to the user.

The present invention represents a significant advance over the technology disclosed in United States Patent Application No. 09/526,169, which is incorporated herein by reference. The present invention extends this technology to cover residential malodors of high intensity including trash odor, urine odor, kitchen odors, and pet litter odor.

After extensive testing, the present inventors surprisingly found that the suppression of residential malodor does not depend on the masking material's odor detection threshold or on the perceived inherent intensity of the masking material. Rather, they found that the suppression of residential malodor could be lessened by use of masking material having a high air diffusion coefficient.

The present invention concerns a composition for suppressing residential malodor, said composition comprising:

a fragrance having at least one perfume material which satisfies one of the following criteria:

contains a phenyl ring moiety and has an air diffusion coefficient of > 5.7,
contains a C-5 ring moiety, which also contains at least 1 carbon which is sp2 hybridized, and has an air diffusion coefficient of > 4.4.

The fragrance contains at least 25% of the perfume material satisfying criteria (1) or (2).

The residential malodor is selected from the group consisting of trashcan odor, pet litter odor, and kitchen odor.

The composition is selected from the group consisting of household cleaners, surfaces cleaners, skin cleaners, deodorant, air fresheners, and deodorizers.

Further, the present invention concerns a method for masking a residential malodor, said method comprising applying to the malodor source a composition containing a fragrance having at least one perfume material which satisfies one of the following criteria:

contains a phenyl ring moiety and has an air diffusion coefficient of > 5.7,
contains a C-5 ring moiety, which also contains at least 1 carbon which is sp2 hybridized, and has an air diffusion coefficient of > 4.4.

The method of the present invention can be used to suppress malodors such as kitchen odors, skin odors, in a solid surface, and pet litter.

The foregoing has outlined rather broadly the more pertinent and important features of the present invention in order that the detailed description of the invention that follows may be better understood, and so that the present contribution to the art can be more fully appreciated. Additional features of the invention that will be described hereinafter form the subject matter of the claims of the invention. It should be appreciated by those skilled in the art that the conception and the specific embodiments disclosed might be readily utilized as a basis for modifying or formulating other liquid formulations for carrying the same purposes of the present invention. It should also be realized by those skilled in the art that such equivalent formulations do not depart from the spirit and scope of the invention as set forth in the appended claims.

The air diffusion coefficient values are expressed in terms of $10^2$ with units of $m^2$/sec. For example, 5.7 is equivalent to 5.7 x $10^{-2}$ $m^2$/sec. Diffusivity depends on molecular size, temperature, pressure, and viscosity of the medium (air). Diffusivity is defined as

$$D = mh/A\ (\Delta d)t$$

Wherein m = mass of substance, h = distance of diffusion, A = cross-sectional area of diffusion, $\Delta d$ = concentration difference over distance h, and t = time.

According to Fick's First Law, the mass flux (J) is proportional to the concentration gradient dC/dz) by the diffusivity of a substance (D).

$$J = (1/A)\ (dm/dt) = D\ (dC/dz)$$

wherein A is the area and dmdt is the flow rate.

Those skilled in the art know that they only can incorporate in the compositions those fragrances that will contribute usefully to the odor of the composition and to the masking of the malodor, either by a smothering effect or by a blending effect.

The present invention concerns a composition for suppressing residential malodor, said composition comprising:

a fragrance having at least one perfume material which satisfies one of the following criteria:

contains a phenyl ring moiety and has an air diffusion coefficient of > 5.7,
contains a C-5 ring moiety, which also contains at least 1 carbon which is sp2 hybridized, and has an air diffusion coefficient of > 4.4.

The fragrance contains at least 25% of the perfume material satisfying criteria (1) or (2).

The residential malodor is selected from the group consisting of urine, trashcan odor, pet litter odor, and kitchen odor such as onion, garlic, seafood, etc.

The composition is selected from the group consisting of household cleaners, surfaces cleaners, deodorant, skin cleaners, air fresheners, and deodorizers.

Further, the present invention concerns a method for masking a residential malodor, said method comprising applying to the malodor source a composition containing a fragrance having at least one perfume material which satisfies one of the following criteria:

contains a phenyl ring moiety and has an air diffusion coefficient of > 5.7,
contains a C-5 ring moiety, which also contains at least 1 carbon which is sp2 hybridized, and has an air diffusion coefficient of > 4.4.

The method of the present invention can be used to suppress malodors such as kitchen odors, skin odors, in a solid surface, and pet litter.

After subjecting a variety of fragrances to a variety of tests, the results are recorded on table for comparative analysis, it was discovered that the ability of a material to mask malodors can be predicted using the following criteria, wherein suitable materials fall into two groups:

Group 1: Materials falling into this category are expected to mask malodors if they contain a phenyl ring moiety and an air diffusion coefficient of > 5.7.

Group 2: Materials falling into this category are expected to mask malodors if they contain a C-5 ring moiety, which also contains at least 1 carbon which is sp2 hybridized and has an air diffusion coefficient of > 4.4.

The inventor found out that it is critical to the present invention that the composition contains at least 25% of materials of Group I and/or Group 2 in order to cover the malodor.

Table 1

| Material | Dragoco Number | Air Diffusion Coefficient | Average Coverage | Phenyl Ring Moiety? | C-5 ring with at least 1 sp2 carbon |
|---|---|---|---|---|---|
| ALLYL CYCLOHEXYL PROPIONATE | 3/011241 | 4.86 | 2.6 | N | N |
| ALLYL HEPTOATE (C-7) | 4/010500 | 5.23 | 2.6 | N | N |
| ANTHER | 3/912100 | 4.86 | 2.6 | Y | N |
| BENZALDEHYDE* | 3/914590 | 7.5 | 3.5 | Y | N |
| CAMPHENE* | 4/726750 | 6.13 | 3.0 | N | N |
| CEDRAMBER | 3/018911 | n/a | 2.3 | N | N |
| CEDROL LIQUID 10% | 3/019281 | 4.25 | 3.3 | N | N |
| CISTULATE 09126 | 3/920335 | n/a | 2.6 | N | N |
| CLONAL | 3/923790 | 4.66 | 3.0 | N | N |
| CRESSANTHER | 3/045062 | n/a | 2.3 | Y | N |
| CYCLOHEXYL ETHANOL | 3/924060 | 6.29 | 2.0 | N | N |

Table 1   (continued)

| Material | Dragoco Number | Air Diffusion Coefficient | Average Coverage | Phenyl Ring Moiety? | C-5 ring with at least 1 sp2 carbon |
|---|---|---|---|---|---|
| DELPHONE, FIRMENICH | 4/916128 | 5.46 | 4.0 | N | Y |
| DIHYDRO TERPINEOL | 4/016236 | 5.47 | 2.0 | N | N |
| DIOLA | 3/926899 | 6.51 | 4.3 | N | N |
| ETHYL ACETOACETATE | 4/910050 | 5.54 | 1.6 | N | N |
| EUCALYPTUS GLOBULUS | 1/913422 | n/a | 2.0 | N | N |
| FLORAZONE | 3/034395 | 4.99 | 3.6 | Y | N |
| FLOROL | 3/934060 | 5.21 | 3.0 | N | N |
| GALAXOLIDE 50 DEP 10% | 4/900551 | 4.07 | 3.0 | N | N |
| GERANYL ACETATE | 3/039991 | 4.78 | 1.0 | N | N |
| GRAPEFRUIT BASE 15.794 B* | 0/115317 | n/a | 1.5 | U | U |
| HEXYL CINNAMIC ALDEHYDE | 3/011371 | 4.45 | 2.3 | Y | N |
| ISO BORNYL ACETATE | 3/943760 | 4.92 | 2.6 | N | N |
| ISO E SUPER | 0/115115 | 4.18 | 3.3 | N | N |
| LILIAL | 4/929325 | 4.7 | 2.6 | Y | N |
| MAYOL | 3/954401 | 5.45 | 2.3 | N | N |
| MELONAL | 3/054211 | 5.79 | 2.0 | N | N |
| METHYL CHAVICOL | 4/131155 | 5.91 | 2.3 | Y | N |
| METHYL IONONE GAMMA | 4/931205 | 4.53 | 3.3 | N | N |
| METHYL NAPTHYL KETONE 10% | 4/933020 | 5.52 | 2.0 | Y | N |
| NEROLIN YARA YARA 10% | 3/959050 | 5.84 | 2.6 | Y | N |
| ORANGE JUICE CARBONYLS* | 4/777119 | n/a | 2.5 | N | N |
| ORANGE OIL FLORIDA | 4/777160 | n/a | 2.3 | U | U |
| PADMA | 3/064461 | 5.71 | 3.3 | Y | N |
| PETIOLE | 4/780162 | n/a | 2.6 | Y | N |
| PHENAFLEUR | 3/964150 | 4.77 | 2.3 | Y | N |
| PHENYL ETHYL ALCOHOL | 3/964540 | 6.76 | 4.3 | Y | N |
| PRENYL ACETATE | 3/026832 | 6.49 | 1.0 | N | N |
| RHUBAFURAN | 4/941135 | n/a | 2.6 | Y | N |
| RHUBOFIX | 3/971450 | n/a | 3.0 | N | N |
| ROSEMARY OIL TUNISIA | 1/510706 | n/a | 2.6 | N | N |

* = tested only at 5 and 38 C

Table 1   (continued)

| Material | Dragoco Number | Air Diffusion Coefficient | Average Coverage | Phenyl Ring Moiety? | C-5 ring with at least 1 sp2 carbon |
|---|---|---|---|---|---|
| SANDRANOL | 3/070540 | 4.44 | 3.3 | N | Y |
| TETRAHYDRO LINALOOL | 4/901360 | 5.33 | 2.6 | N | N |
| TONALID 10% | 3/976500 | | 2.0 | N | N |
| TRIDECENE-2-NITRILE | 3/076791 | 4.32 | 3.0 | N | N |
| VELOUTONE | 4/949088 | 4.59 | 3.0 | N | Y |
| YLANAT | 3/084021 | 4.79 | 2.3 | N | N |
| YLANAT ORTHO | 3/084027 | 4.77 | 2.6 | N | N |
| YSAMBER K | 3/055120 | n/a | 2.0 | N | N |

As it can be seen from Table 1, fragrance materials that satisfy one of the following criterias:

Group 1:   contains a phenyl ring moiety and an air diffusion coefficient of > 5.7; or

Group 2:   contains a C-5 ring moiety, which also contains at least 1 carbon which is sp2 hybridized and has an air diffusion coefficient of > 4.4.

Cover the malodor better than those that do not meet any of the criterias.

**Description of the testing procedure:**

**Example 1 (Kitchen Odor)**

1) A garlic solution having 75% of water was blended for 2 minutes and 0.02 grams of the solution were weighed out.
2) The panelist washed each forearm with unfragranced soap.
3) The garlic solution was applied to each of the panelist's washed forearms, and rubbed in for 30 seconds and then the forearm was rinsed for 15 seconds with water.
4) A liquid soap containing 1% of the odor masking fragrance, which meets one of the two criteria, according to the present invention, was applied to one of the forearms and rinsed off for 15 seconds.
5) A liquid soap containing a fragrance that does not include the raw material that meets one of the two criteria, according to the present invention, was applied to the forearm and rinsed off after 15 seconds.
6) The skin was dry, and the panelist evaluated the odor and judged which arm had the least malodor residue.

**Example 2 (Kitchen Odor)**

1) An onion solution having 75% of water was blended for 2 minutes, and 0.02 grams of the solution were weighed out.
2) The panelist washed each forearm with unfragranced soap.
3) The onion solution was applied to each of the panelist's washed forearms, and the solution was rubbed in for 30 seconds and then rinsed for 15 seconds with water.
4) A liquid soap containing 1% of the odor masking fragrance, which meets one of the two criteria according to the present invention was applied to one of the forearms and rinsed off for 15 seconds.
5) A liquid soap containing a fragrance that does not include a fragrance, which meets one of the two criteria, according to the present invention, was applied to the other forearm and rinsed off after 15 seconds.
6) The skin was dry, and the panelist evaluated the odor and judged which arm had the least malodor residue.

**Example 3 (Kitchen Odor)**

1) A fish solution having 80% of water was blended for 2 minutes, and 0.02 grams of the solution was weighed out.
2) The panelist washed each forearm with unfragranced soap.
3) The fish solution was applied to each of the panelist's washed forearms, and the odor was rubbed in for 30 seconds and then rinsed for 15 seconds with water.
4) A liquid soap containing 1% of the odor masking fragrance, which meets one of the two criteria, according to the present invention, was applied to one of the forearms and rinsed off for 15 seconds.
5) A liquid soap containing a fragrance that does not include a fragrance, which meets one of the two criteria, according to the present invention, was applied to the other forearm and rinsed off after 15 seconds.
6) The skin was dry, and the panelist evaluated the odor and judged which arm had the least malodor residue.

**Example 4 (Trash malodor)**

The following formulation was used to simulate trash malodor (source: US General Services Admin. Federal Supply)
Units

| | |
|---|---|
| Diacetyl | 0.4 |
| Puradine | 0.43 |
| Diallyl sulfide | 0.96 |
| Dimethyl sulfide | 4.04 |
| Heptaldehyde | 0.39 |
| **Propionic acid** | **3.76** |
| **Acetic acid** | **0.06** |

Preparation of malodor and fragrances:

1) A 10% EDT was prepared with the kitchen malodor oil and Alcohol SD 39.
2) 1 gram of the 10% kitchen malodor was weighed into a 4 oz. glass jar and a cotton cosmetic pad added and soaked in the solution.
3) After 10 minutes of air drying, this was capped and sealed with parafilm
4) 0.3 of these were prepared for each test.

Evaluation procedure:

A 25% EDT was prepared for the fragrance oil in Alcohol SD 39. A trash container was lined with a plastic bag. The fragrance EDT was sprayed on 4 sides inside of the plastic bag and evaluated after 10 minutes

**Example 5 (Cat Litter malodor) Urine odor**

Syloid 244 (manufactured by W.R. Grace), which includes fragranced beads with traditional clay litter, was used:

1) 1.5 g of Syloid 244 and 0.5 g fragrance material, which does not meet one of the criteria of the present invention, were mixed and stored in the refrigerator for 24 hours.
2) 1.5 g of Syloid 244 and 0.5 g fragrance material, which meet one of the criteria, according to the present invention, were mixed and stored in the refrigerator for 24 hours.
3) For each fragrance to be evaluated, 3 sets of cat litter are prepared:

1,000 g of clay-type cat litter in a plastic tray with a cover;
1,000 g of unfragranced clay-type cat litter and 2 g of Syloid fragranced beads (fragrance material do not meet one of the criteria of the present invention) are mixed and placed in a plastic tray with a cover; and

1,000 g of unfragranced clay-type cat litter and 2 g of Syloid fragrance beads (fragrance material that meet one of the criteria of the present invention) are mixed and placed in a plastic tray with a cover.

4) 5 ml of cat urine were placed in the center of each tray of cat litter and evaluated immediately after 10 minutes.

**Example 6 (Cat Litter malodor)**

1) Syloid 244 (manufactured by W.R. Grace), which includes fragranced beads with clumping cat litter, was used.
2) 1.5 g of Syloid 244 and 0.5 g fragrance material, which do not meet one of the criteria of the present invention were mixed and stored in the refrigerator for 24 hours.
3) 1.5 g of Syloid 244 and 0.5 g fragrance material, which meet one of the criteria of the present invention, were mixed and stored in the refrigerator for 24 hours.
4) For each fragrance to be evaluated, 3 sets of cat litter are prepared:

1,000 g of unfragranced clumping cat litter in a plastic tray with a cover;
1,000 g of unfragranced clumping cat litter and 0.7g of Syloid fragranced beads are mixed and placed in a plastic tray with a cover; and
1,000 g of unfragranced clumping cat litter and 0.7g of Syloid fragrance containing maskant beads are mixed and placed in a plastic tray with a cover.

5) 5 ml of cat urine were placed in the center of each tray of cat litter and evaluated immediately after 10 minutes.

**Results of panel testing:**

For each malodor, 27 - 32 panelists were polled.
The test was conducted by asking the member of the panel to smell the control and two products of which one of them was the control that acts as a blind control.
The panelists were told that one was the control and the other one was a test fragrance.
N represents the number of panelists.

**Kitchen malodor test (Onion malodor results)**

| N=30 | Variant | Blind Control |
|---|---|---|
| | 407 | 883 |
| **In comparison to the control, number of panelists who responded that the odor was:** | 3.2 | 2.5 |
| 1 The same as the control | 17 | 27 |
| 2 | 13 | 23 |
| 3 Moderately different than the control | 30 | 33 |
| 4 | 17 | 7 |
| 5 Very different than the control | 23 | 10 |
| | | |
| **In comparison to the control, number of panelists who responded that the sample smelled:** | 3.8 | 3.0 |
| 1 Much worse than the control | 3 | 10 |
| 2 | 13 | 20 |

(continued)

| N=30 | Variant | Blind Control |
|---|---|---|
|  | **407** | **883** |
| 3 About the same as the control | 20# | 43 * |
| 4 | 27 | 17 |
| 5 Much better than the control | 37* | 10# |
|  |  |  |
| *Net Worse* | *26* | *30* |
| *Net Better* | *64** | *27#* |
|  |  |  |
| **Rating of the degree to which the panelist judged this sample to reduce the malodor smell in the control:** | 4.4 | 3.5 |
| 1 Not At All | 0# | 17* |
| 2 | 3 3 |  |
| 3 Moderately | 17 | 30 |
| 4 | 20 | 10 |
| 5 Very Much | 60 | 40 |
|  |  |  |
| **Overall sample preference:** |  |  |
| Sample | 69* | 28# |
| Control | 31# | 66* |
| Equal | 0 | 7 |

* indicates significantly higher score than other sample (@ 90% confidence level)

# indicates significantly lower score than other sample (@ 90% confidence level)

**Kitchen malodor test (Garlic malodor coverage)**

| N=28 | Variant | Blind Control |
|---|---|---|
|  | **513** | **947** |
| **In comparison to the control, number of panelists who responded that the odor was:** | 3.2 | 2.8 |
| 1 The same as the control | 7 | 21 |
| 2 | 14 | 18 |
| 3 Moderately different than the control | 43 | 39 |
| 4 | 21* | 4# |
| 5 Very different than the control | 14 | 18 |
|  |  |  |

* indicates significantly higher score than other sample (@ 90% confidence level)

# indicates significantly lower score than other sample (@ 90% confidence level)

(continued)

| N=28 | Variant | Blind Control |
|---|---|---|
| | 513 | 947 |
| **In comparison to the control, number of panelists that responded that the sample smelled:** | 3.4 | 2.8 |
| 1 Much worse than the control | 14 | 14 |
| 2 | 11# | 29* |
| 3 About the same as the control | 18 | 32 |
| 4 | 36 | 18 |
| 5 Much better than the control | 21 | 7 |
| | | |
| *Net Worse* | *25* | *43* |
| *Net Better* | *57** | *25#* |
| | | |
| **Rating of the degree to which the panelist judged this sample to reduce the malodor smell in the control:** | 3.3 | 2.9 |
| 1 Not At All | 7 | 14 |
| 2 | 18 | 21 |
| 3 Moderately | 36 | 36 |
| 4 | 18 | 21 |
| 5 Very Much | 21 | 7 |
| | | |
| **Overall sample preference:** | | |
| Sample | 64* | 41# |
| Control | 32 | 52 |
| Equal | 4 | 7 |

* indicates significantly higher score than other sample (@ 90% confidence level)

# indicates significantly lower score than other sample (@ 90% confidence level)

**Kitchen malodor test (Fish malodor coverage)**

| N=27 | Variant | Blind Control |
|---|---|---|
| | 372 | 654 |
| **In comparison to the control, number of panelists who responded that the odor was:** | 3.0 | 2.3 |
| 1 The same as the control | 15# | 37* |
| 2 | 22 | 33 |

* indicates significantly higher score than other sample (@ 90% confidence level)

# indicates significantly lower score than other sample (@ 90% confidence level)

(continued)

| N=27 | Variant | Blind Control |
|---|---|---|
| | 372 | 654 |
| 3 Moderately different than the control | 26* | 7# |
| 4 | 19 | 7 |
| 5 Very different than the control | 19 | 15 |
| | | |
| **In comparison to the control, number of panelists who responded that the sample smelled:** | 3.8 | 3.4 |
| 1 Much worse than the control | 0 | 0 |
| 2 | 11 | 11 |
| 3 About the same as the control | 26# | 56* |
| 4 | 37 | 19 |
| 5 Much better than the control | 26 | 15 |
| | | |
| *Net Worse* | *11* | *11* |
| *Net Better* | *63** | *34#* |
| | | |
| **Rating of the degree to which the panelist judged this sample to reduce the malodor smell in the control:** | 4.4 | 4.3 |
| 1 Not At All | 0 | 0 |
| 2 | 0 0 | 0 |
| 3 Moderately | 19 | 7 |
| 4 | 19# | 48* |
| 5 Very Much | 63 | 44 |
| | | |
| **Overall sample preference:** | | |
| Sample | 82* | 52# |
| Control | 19# | 41* |
| Equal | 0 | 7 |

\* indicates significantly higher score than other sample (@ 90% confidence level)

\# indicates significantly lower score than other sample (@ 90% confidence level)

**Cat litter malodor coverage (Traditional clay cat litter)**

| N=28 | Variant | Blind Control |
|---|---|---|
| | 340 | 751 |
| **In comparison to the control, number of panelists that responded that the odor was:** | 3.0 | 2.7 |

(continued)

| N=28 | Variant | Blind Control |
|---|---|---|
| | 340 | 751 |
| 1 The same as the control | 14 | 27 |
| 2 | 11 | 19 |
| 3 Moderately different than the control | 39 | 23 |
| 4 | 29 | 19 |
| 5 Very different than the control | 7 | 12 |
| | | |
| **In comparison to the control, number of panelists that responded that the sample smelled:** | 3.4 | 3.2 |
| 1 Much worse than the control | 4 | 4 |
| 2 | 29 | 23 |
| 3 About the same as the control | 14# | 42* |
| 4 | 32 | 15 |
| 5 Much better than the control | 21 | 15 |
| | | |
| *Net* Worse | 33 | 27 |
| *Net Better* | 53* | 30# |
| | | |
| **Rating of the degree to which the panelist judged this sample to reduce the malodor smell in the control:** | 3.1 | 3.1 |
| 1 Not At All | 11 | 8 |
| 2 | 11 | 27 |
| 3 Moderately | 46 | 31 |
| 4 | 18 | 15 |
| 5 Very Much | 14 | 19 |
| | | |
| **Overall sample preference:** | | |
| Sample | 57 | 54 |
| Control | 43 | 39 |
| Equal | 0 | 8 |

* indicates significantly higher score than other sample (@ 90% confidence level)

# indicates significantly lower score than other sample (@ 90% confidence level)

**Cat litter malodor (Encapsulated fragrance in cat litter)**

| N=30 | Variant | Blind Control |
|---|---|---|
| | 521 | 837 |
| **In comparison to the control, number of panelists who responded that the odor was:** | 2.9 | 2.7 |
| 1 The same as the control | 23 | 20 |
| 2 | 7# | 23* |
| 3 Moderately different than the control | 40 | 33 |
| 4 | 13 | 10 |
| 5 Very different than the control | 17 | 13 |
| **In comparison to the control, number of panelists that responded that the sample smelled:** | 3.3 | 2.6 |
| 1 Much worse than the control | 10 | 20 |
| 2 | 13 | 30 |
| 3 About the same as the control | 23 | 30 |
| 4 | 40* | 13# |
| 5 Much better than the control | 13 | 7 |
| *Net Worse* | 23# | 50* |
| *Net Better* | 53* | 20# |
| **Rating of the degree to which the panelist judged this sample to reduce the malodor smell in the control:** | 2.6 | 2.3 |
| 1 Not At All | 17# | 37* |
| 2 | 33 | 30 |
| 3 Moderately | 30* | 10# |
| 4 | 13 | 13 |
| 5 Very Much | 7 | 10 |
| **Overall sample preference:** | | |
| Sample | 60* | 27# |
| Control | 40# | 67* |
| Equal | 0 | 7 |

\* indicates significantly higher score than other sample (@ 90% confidence level)

# indicates significantly lower score than other sample (@ 90% confidence level)

**Trash malodor coverage**

| N=32 | Variant | Blind Control |
|---|---|---|
| | **337** | **502** |
| **In comparison to the control, number of panelists who responded that the odor was:** | 3.7 | 2.6 |
| 1 The same as the control | 9 | 22 |
| 2 | 0# | 13* |
| 3 Moderately different than the control | 31# | 53* |

\* indicates significantly higher score than other sample (@ 90% confidence level)

# indicates significantly lower score than other sample (@ 90% confidence level)

(continued)

| N=32 | Variant | Blind Control |
|---|---|---|
| | 337 | 502 |
| 4 | 34* | 6# |
| 5 Very different than the control | 25* | 6# |
| | | |
| **In comparison to the control, number of panelists who responded that the sample smelled:** | 3.8 | 2.8 |
| 1 Much worse than the control | 3 | 9 |
| 2 | 16 | 28 |
| 3 About the same as the control | 13# | 38* |
| 4 | 38 | 25 |
| 5 Much better than the control | 31* | 0# |
| | | |
| *Net Worse* | *19* | *37* |
| *Net Better* | *69** | *25#* |
| | | |
| **Rating of the degree to which the panelist judged this sample to reduce the malodor smell in the control:** | 3.3 | 2.5 |
| 1 Not At All | 3# | 16* |
| 2 | 22 | 38 |
| 3 Moderately | 22 | 28 |
| 4 | 44* | 16# |
| 5 Very Much | 9 | 3 |
| | | |
| **Overall sample preference:** | | |
| Sample | 72* | 44# |
| Control | 28# | 56* |
| Equal | 0 | 0 |

\* indicates significantly higher score than other sample (@ 90% confidence level)

\# indicates significantly lower score than other sample (@ 90% confidence level)

The previous results show that there was a significant preference for the fragrance containing a perfume material, which meets one of the criteria according to the present invention.

Although this invention has been described in its preferred form with a certain degree of particularity with respect to a method for determining perfumes to be used to mask ammonia in a hair treatment composition, it is understood that the present disclosure of the preferred form has been made only by way of example, and that numerous changes in the details of structures and composition of the product may be resorted to without departing from the spirit and scope of the invention.

**Claims**

1. Method for masking a residential malodor, said method comprising applying to the malodor source a composition containing a fragrance having at least one perfume material which satisfies one of the following criteria:

> (1) contains a phenyl ring moiety and has an air diffusion coefficient of > 5.7,
> (2) contains a C-5 ring moiety, which also contains at least 1 carbon which is sp2 hybridized, and has an air diffusion coefficient of > 4.4.

2. Method according to claim 1, wherein said composition contains at least 25% of the perfume material satisfying criteria (1) or (2).

3. Method according to any of claims 1 - 2, wherein the residential malodor is selected from the group consisting of trashcan odor, pet litter odor, urine odor and kitchen odor.

4. Method according to claim 3, wherein the residential malodor is kitchen odor selected from the group consisting of garlic, onion, and fish.

5. Composition for suppressing residential malodor, said composition comprising:

a fragrance having at least one perfume material which satisfies one of the following criteria:

> (1) contains a phenyl ring moiety and has an air diffusion coefficient of > 5.7,
> (2) contains a C-5 ring moiety, which also contains at least 1 carbon which is sp2 hybridized, and has an air diffusion coefficient of > 4.4.

6. Composition according to claim 5, wherein said fragrance contains at least 25% of the perfume material satisfying criteria (1) or (2).

7. Composition according to any of claims 5 - 6, wherein said composition is selected from the group consisting of household cleaners, surfaces cleaners, skin cleaners, deodorant, air fresheners, and deodorizers.

8. Method of controlling malodors on skin comprising:

applying to the skin a composition comprising:

a fragrance having at least one perfume material which satisfies one of the following criteria:

> (1) contains a phenyl ring moiety and has an air diffusion coefficient of > 5.7,
> (2) contains a C-5 ring moiety, which also contains at least 1 carbon which is sp2 hybridized, and has an air diffusion coefficient of > 4.4.

9. Method of controlling malodors on skin according to claim 8, wherein said fragrance contains at least 25% of the perfume material satisfying criteria (1) or (2).

10. Method for suppressing malodor in a solid surface, the method comprising the step of applying onto said solid surface, a composition comprising a fragrance having at least one perfume material which satisfies one of the following criteria:

> (1) contains a phenyl ring moiety and has an air diffusion coefficient of > 5.7, or
> (2) contains a C-5 ring moiety, which also contains at least 1 carbon which is sp2 hybridized, and has an air diffusion coefficient of > 4.4.

11. Method according to claim 10, wherein the solid surface is selected from the group consisting of trashcan, diaper, and kitchen counter.

12. Method for suppressing malodor in a pet litter, the method comprising the steps of:

contacting the pet litter with a composition containing a fragrance having at least one perfume material which satisfies one of the following criteria:

(1) contains a phenyl ring moiety and has an air diffusion coefficient of > 5.7,

(2) contains a C-5 ring moiety, which also contains at least 1 carbon which is sp2 hybridized, and has an air diffusion coefficient of > 4.4.

**13.** Use of a perfume material which satisfies one of the following criteria:

(1) contains a phenyl ring moiety and has an air diffusion coefficient of > 5.7,

(2) contains a C-5 ring moiety, which also contains at least 1 carbon which is sp2 hybridized, and has an air diffusion coefficient of > 4.4.

for masking a residential malodor or controlling malodor on skin or suppressing malodor in a solid surface.

**European Patent Office**

## EUROPEAN SEARCH REPORT

Application Number

EP 03 01 8903

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 195 099 A (TAKASAGO INT. CORP.) 10 April 2002 (2002-04-10) * paragraph [0029] * * paragraph [0056] - paragraph [0062]; claims 1-14 * --- | 1-12 | A61L9/01 C11B9/00 |
| X | DE 100 51 816 A (HENKEL KGAA) 2 May 2002 (2002-05-02) * paragraph [0017] - paragraph [0018] * --- | 1-12 | |
| X,P | WO 02 085420 A (QUEST INTERN.) 31 October 2002 (2002-10-31) * page 3, paragraph 3; claims 1-11; example 1 * ----- | 1-12 | |

TECHNICAL FIELDS SEARCHED (Int.Cl.7)

A61L
C11B

The present search report has been drawn up for all claims

| Place of search | Date of completion of the search | Examiner |
|---|---|---|
| MUNICH | 21 October 2003 | Luethe, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 03 01 8903

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2003

| Patent document cited in search report | | Publication date | Patent family member(s) | | Publication date |
|---|---|---|---|---|---|
| EP 1195099 | A | 10-04-2002 | EP<br>JP | 1195099 A2<br>2002113080 A | 10-04-2002<br>16-04-2002 |
| DE 10051816 | A | 02-05-2002 | DE<br>AU<br>WO | 10051816 A1<br>2061102 A<br>0232471 A1 | 02-05-2002<br>29-04-2002<br>25-04-2002 |
| WO 02085420 | A | 31-10-2002 | WO | 02085420 A1 | 31-10-2002 |

For more details about this annex : see Official Journal of the European Patent Office, No. 12/82